# EUROPÄISCHE PATENTSCHRIFT

(11) **EP 0 649 652 B1**
(45) Veröffentlichungstag und Bekanntmachung des Hinweises auf die Patenterteilung: **02.06.1999**
(21) Anmeldenummer: 94116072.3
(22) Anmeldetag: 12.10.1994
(51) Int. Cl.: A61K 9/70

(54) **Verwendung grenzwertig wirksamer Arzneiformen mit Betablockern**
Use of minimal-dose beta-blocker preparations
Utilisation des compositions des bêta-antagonistes à dosage minimale

(30) Priorität: 13.10.1993 DE 4334919
(43) Veröffentlichungstag der Anmeldung: 26.04.1995
(73) Patentinhaber: APL - American Pharmed Labs, Inc., Englewood Cliffs, N.J. 07632 (US)
(72) Erfinder: Liedtke, Dr. Rainer K., D-82027 Grünwald bei München (DE)
(74) Vertreter: Bühling, Gerhard, Dipl.-Chem.

(56) Entgegenhaltungen:
- EP-A- 0 499 662
- AM. J. CARDIOL., Bd.58, Nr.3, 1986 Seiten 273 - 278 J. MORGANROTH ET AL. 'Effectiveness of low-dose nadolol for ventricular arrhythmias.'
- CLIN. THER., Bd.15, Nr.5, 1993 Seiten 779 - 787 P.K. ZACHARIAH ET AL. 'Low-dose bisoprolol/hydrochlorothiazide: an option in first-line antihypertensive treatment.'

## Beschreibung

Die Erfindung betrifft die Verwendung grenzwertig wirksamer Arzneiformen mit hydrophilen Betablockern, zur Kurzzeit-Therapie transienter funktioneller Herz-Kreislauf-Symptomatiken.

Es ist bekannt, daß die chemische Gruppe der Betarezeptorenblocker in der Langzeittherapie von manifesten Herz-Kreislauferkrankungen wie Hypertonie und Angina pectoris zum Standard gehört. Das Anwendungspektrum der Betablocker beinhaltet zudem indikationsmäßig noch u.a. Hyperkinetisches Herzsyndrom, Migräne, Tremor, Glaukom, Angstsyndrome, Entzugssyndrome (e.g. H.Lydtin, P. Trenkwalder, Neue Indikationen für die Therapie mit Betarezeptorenblockern in: Betarezeptorenblocker, G. Fischer, Stuttgart, 1991, S. 58ff.)

Herz-Kreislauf Erkrankungen lassen sich diagnostisch prinzipiell unterteilen in Erscheinungsformen mit pathologischen Manifestationen, z.B. Angina pectoris auf Basis einer manifesten koronaren Herzkrankheit, und in Symptomatiken mit nur transient auftretenden pathologischen Erscheinungen ohne ausgeprägtes morphologisches Substrat, z.B. situationsbedingt reaktive Beschleunigungen der Herzfrequenz durch exogene oder endogene physische oder psychische Stressoren. Diesem Bereich, im weiteren Sinne, auch zuzurechnen sind z.B. transiente kardiale Ischämien, sogenannte stumme myokardiale ischämische Episoden, die seitens der Patienten nicht bemerkt, aber im EKG auffindbar sind. Transiente Erscheinungen dieser Art werden auch als funktionelle oder vegetative Symptomatiken bezeichnet und basieren primär auf situativ inadäquaten Reaktionen des sympathischen Nervenstems bzw. der von diesem freigesetzten adrenergen Neurotransmitter.

Diese diagnostische Unterteilung beinhaltet aber. prinzipiell auch therapeutisch unterschiedliche Ziele und verlangt damit unterschiedliche Erfordernisse für die Betablocker-therapie, die im wesentlichen zu berücksichtigen hat, daß es sich bei den vegetativen Patienten um organisch weitestgehend Gesunde handelt. Daher gehören hierzu u.a. Unterschiede in der erforderlichen Behandlungsdauer, dem unterschiedlichen Grad an erforderlicher therapeutischer Effektausprägung, unterschiedlichen Dosierungserfordernissen sowie auch insgesamt eine unterschiedliche Bewertung der diesbezüglich therapeutischen Nutzen/Risiko Relation.

Die derzeit verfügbaren Arzneiformen wie auch Dosierungsschemata mit Betablockern tragen diesen therapeutisch unterschiedlichen Erfordernissen in keiner Weise Rechnung. So werden auch für die Therapie von transienten vegetativen Symptomatiken undifferenziert die identischen Arzneiformen mit Betablockern eingesetzt wie sie zur Langzeittherapie manifester Herz-Kreislauf Erkrankungen konzipiert sind. Es wird lediglich versucht, dies äußerlich etwas anzupassen, z.B. durch Empfehlungen - in Rahmen des für die manifesten und chronischen Krankheitsbilder bereits vorgegeben verfügbaren - Dosis-Spektrums ggfs. deren untere Dosisbereiche einzusetzen. Wobei dies aber auch dort noch mit den gleichen Dosierfrequenzen erfolgt.

Ein Einsatz der Arzneiformen mit Betablockern, wie er in der Therapie chronischer Herz-Kreislauf Patienten erfolgt, ist aber bei symptomatischen Patienten mit transienter Herz-Kreislauf-Reaktionen grundsätzlich eine pharmakodynamisch unnötige Überdosierung, mit einem erhöhten Risiko von unerwünschter Wirkungen.

Das Gesamtspektrum derzeit verfügbarer und für Langzeittherapie konzipierter Arzneiformen mit Betablockern setzt u.a. die physische Gesamtleistungskapazität herab und beeinflusst z.B. u.a. den Lipid- und Glukose-Metabolismus (e.g. M. Wickelmayr et al., Glucose Metabolism and β-Blockers, Diuretics and Calcium-Antagonists, pp 29-33, in: Effects of Antihypertensive treatment on Glucose Metabolism, International Symposium Bühlerhöhe 1988, Thieme, Stuttgart-New York 1990). Da diese Arzneiformen pharmakokinetisch schon initial vollständige Absättigung adrenerger Rezeptoren bewirken, führt in der Regel bereits deren Erstdosis durch kompetitive Verdrängung endogener Katecholamine zu einer langanhaltenden Änderung auch der Ruheparameter des Herz-Kreislaufs. Dies ist für transiente vegetative Symptomatiken aber nicht nur unnötig, sondern eher schädlich.

Als Folge müssen Patienten mit transienten Symptomatiken bei Gabe dieser üblichen Arzneiformen aus dieser Art Betablocker-Therapie auch erst wieder stufenweise herausdosiert werden, um nicht der Gefahr von kardiovasculären Absetzphänomenen, sogenannten Rebounds, wie z.B. reaktiven Steigerungen der Herzfrequenz, zu unterliegen. Dies zwingt daher zu einer längeren Anwendung der Betablocker als es für diese transienten Symptomatiken therapeutisch erforderlich ist.
Vegetative Krankheitsbilder sind sowohl alle akut überschießenden Reaktionen des sympathischen Nervensystems auf physische und psychische Stressoren, aber auch vegetative Einzelsymptomatiken, die in verschiedene Syndrome eingebettet sind. Zu letzerem können z.B. u.a. auch Schlafstörungen gehören. Diese gravierend belastenden vegetativen Symptomatiken werden aber, durch die ungeeigneten Arzneiformen, einer Betablockertherapie bisher vorenthalten und daher mit anderen Therapien angegangen. So erfolgt derzeit die primäre Therapie von Schlafstörungen mit zentralnervös wirksamen Arzneistoffen, überwiegend aus der chemischen Gruppe der Benzodiazepine, wie auch mit Derivaten der Barbitursäure. Solche Behandlungen zeigen ganz wesentliche Nachteile. Die Sedierungseffekte sind nicht nur ausgeprägt sondern reichen auch, als Überhangeffekte, deutlich in Tagesphasen körperlicher Aktivität. Hierbei kommt es u.a. zu starker Tagesschläfrigkeit und Verminderung operativer Reaktionsfähigkeit und Aufmerksamkeit. Diese Stoffe verfügen zudem über ein erhebliches Abhängigkeitspotential. Absetzen nach wiederholter Gabe löst häufig unerwünschte Wirkungen aus und vegetative Störungen werden sogar noch verstärkt, was die Patienten dann mit weiterer Fortsetzung dieser Therapie verhindern wollen.

Ein Einsatz der Betablocker für eine primäre Therapie von Schlafstörungen gibt es bisher nicht. Dies ist auch nicht naheliegend, da bei den derzeit eingesetzten Arzneiformen mit Betablockern, Schlafstörungen und Alpträume sogar zu den unerwünschten Wirkungen dieser Therapie gehören. Diese unerwünschten Begleitfolgen sind aber u.a. auf die zu stark persistierende Herz-Kreislauf Effekte der üblichen Arzneiformen mit Betablockern zurückzuführen. Insbesondere die lipohilen Betablocker, die stärker die ZNS-Barriere permeieren führen zu besonders ausgeprägten Schlafstörungen (e.g. A. Westerlund, Central Nervous System Side Effects with Hydrophilic and Lipophilic Betablockers, Eur. J. Clin. Pharmacol. (1985) 23 (Suppl) 73-76).

Solche unerwünschten Nebeneffekte lassen sich auch nicht mit üblichen Retard-Arzneiformen von Betablockern verbessern, die z.B. Einmal-täglich Gaben ermöglichen sollen.

Vielmehr wird dies mit solchen Retardformen nur noch weiter verschlechtert, da diese, aus pharmakokinetischen Gründen, noch höher dosiert sein müssen, um eine verlängerte Persistenz der Herz-Kreislauf-Effekte zu erzielen. Effektverlängerungen der gegenüber Betablockern empfindlicheren vegativen Parameter, insbesondere auf die kardiale Chronotropie, sind aber für vegetative Symtomatiken unnötig. So ist die, nur aus der Plasmakonzentration analytisch ermittelte pharmakokinetische Kalbwertszeit der Betablocker weit kürzer als deren pharmakodynamische Wirkdauer. Die nachweisbaren Herzfrequenzreduktionen persistieren weit über die pharmakokinetischen Parameter hinaus, ohne daß für die vegetativen Parameter hieraus eine therapeutische Konsequenz gezogen wurde.

Verstreute Angaben der Literatur weisen darauf hin, daß neben den zentralnervösen Ursachen auch periphere Faktoren in Schlafstörungen involviert sein können. So fallen verschiedene Schlafstörungen deutlich mit transienten vegetativen Reaktionen zusammen. Bei schlechteren Schläfern ließ sich, neben zentralnervös bedingter erhöhter Körpertemperatur, auch eine erhöhte Sekretion der Katecholamine Adrenalin und Noradrenalin feststellen (K. Adam, in: I. Hindmarch, H. Ott, Th. Roth (Edits), Sleep, Benodiazepines and Performance, Springer, Berlin, 1984, 44-53). Vergleichbare Vorgänge scheinen auch im Rahmen des weiblichen Klimakteriums vorzuliegen. So gehen in dieser, durch zahlreiche vegetative Funktionsstörungen gekennzeichneten Phase auch erheblich erhöhte Schlafstörungen einher, insbesondere auch im Rahmen der charakeristischen Hot Flush Anfälle auftretend (e.g. D. Sturdee, M. Brincat in: J. Studd, M. Whitehead (Edits), The Menopause, Blackwell, Oxford, 1988, S. 24-42).

Am.J.Cardiol 58(1986)273 beschreibt eine konventionelle Untersuchung zur individuellen Titrierung des Betablockers Nadolol zur Ermittlung von Mindestdosierungen im Rahmen der spezifischen Arrythmie-Indikaton vorzeitiger ventrikulärer Komplexe und fand keine Beziehung zwischen pharmakokinetischen Cmin Konzentrationen und prozentualer Reduktion der ventrikulären vorzeitigen Komplexe. Die Studie schliesst daher nur mit allgemeinen Betonung der Notwendigkeit einer Wahl individueller Dosen dieses Betablockers für eine Kontrolle ventrikulärer Arrythmien. Clinical Therapeutics 15(1993)779 beschreibt eine Studie für eine Niedrigdosieung des Betablockers Bisoprolol zur Therapie essentieller Hypertonie. In diesem Falle wird die Niedrigdosierung des Betablockers durch ein pharmakologisches Prinzip herbeigeführt, die seit langem bekannte Kombination mit einem synergistisch wirksamen Diuretikum (Hydrochlorothiazid). Ein Vorgehen mit solch einem zweiten Wirkprinzip wäre für transiente vegetative Symptome ohne jeden pharmakologischen Sinn. Zudem verhinderte es jede Aussage über den Dosismindestbereich des Betablockers als Einzelstoff bei transienten cardiovaculären Ereignissen.

Adrenerg induzierte kardiale Erscheinungen wie die Tachycardie scheinen auch Angstanfälle zu verstarken. Das Auftreten transienter Angstsymptome nach experimenteller adrenerger Stimulation bei gesunden Probanden, z.B. durch intravenöses Isoprenalin, ist in der Humanpharmakologie bekannt.

Aus den vorgenannten Aspekten scheint daher eine kardiale, temporär angreifende Reduktion überschießender adrenerger Stimulationen auch als ein - für Schlafstörungen bisher nicht eingesetztes - Prinzip möglich. Angriffspunkt ist hierbei die Blockade von zentralnervös bedingten, den Schlaf störenden Stressfaktoren. Dies erfolgt insbesondere über die, für Betablocker besonders empfindlichen, chronotropen adrenergen kardialen Rezeptoren. Der durch das Zentralnervensystem ausgelöste reaktive Anstieg der Herzfrequenz wird hiermit unterbunden und gleichzeitig unterbleibt ein negatives Reinforcement auf das Zentralnervensystem durch Blockade der kardialen Rückkopplungsreaktion. Dies führt daher zur Unterbrechung eines sich zentralnervös-kardial aufschaukelnden Circulus vitiosus.

Für eine geeignete Therapie transienter vegetativer Symptomatiken mit Betablockern sind bei den funktionellen Störungen jedoch nur Reduktionen unphysiologischer adrenerger Stimulationseffekte sinnvoll. Unnötig und unerwünscht sind demgegenüber nach Abklingen dieser temporären Situationen noch weiter persistierende Dauereffekte der Betablocker auf das Herz-Kreislauf-System, insbesondere persistierende Beeinflussungen von physiologischen Basiswerten des Herz-Kreislauf-Systems. Diese machen sich nur leistungsmindernd bemerkbar und beeinflussen die Lebensqualität negativ. Pharmakologisch ist daher allein eine solcherart "Stand-by" Situation erforderlich, bei der sich der pharmakodynamische Effekt der Betablocker-Arzneiform nur unter Stimulationsbedingungen auswirkt.

Im Rahmen verschiedener klinischer Studien, die mit einem Transdermalsystem mit dem Betablocker Mepindolol in Angina pecrois-Patienten durchgeführt wurden, konnten wir aber bei diesen Patienten Konzentrationen des Betablockers im Serum nachweisen, die um einen Faktor von über 5 niedriger lagen, als dies mit den üblichen therapeutischen Dosen bei üblicher oraler Applikation bei diesem Betablocker der Fall ist. Mit diesen außergewöhnlich niedrigen Wirkstoffkonzentrationen fanden sich differenzierte Beeinflussungen der Herzfrequenz. Es zeigte sich, was mit mit kontinuierlichen 24 Stunden-EKGs objektiviert wurde, daß sich zwar die maximalen Anstiege der Herzfrequenzen signifikant senkten, die unteren Ruhefrequenzen jedoch unverändert blieben. Dies ging auch mit einer signifikanten Abnahme stummer mykokardialer ischämischer Episoden dieser Patienten einher, somit einer Verbesserung von deren kontinuierlicher Herzdurchblutung. Diese Effekte liessen sich dabei auch für noch weiter verkürzte Anwendungen, z.B 12-stündige Anwendungen, reproduzieren (e.g. J. Bonelli, F. Gazo, P. Kirsch, R. K. Liedtke, Int. J. Clin. Pharmacol. Therap. Toxicol. 29, 425-430 (1991).

Somit besteht auch eine Möglichkeit, funktionelle und transiente Stimulationsphasen des adrenergen Systems auch kurzzeitig anzugehen ohne hierbei die basale vegetative Homöostase des Herz-Kreislauf Systems zu beeinflussen. Bei Einsatz aller derzeit therapeutisch eingesetzten üblichen Arzneiformen mit Betablockern finden sich demgegenüber stets, schon bei kreislaufgesunden Probanden, persistierende und signifikante Senkungen der physiologischer Ruhewerte des Herz-Kreislaufs, was mit einer allgemeinen Reduktion der physischen Leistungskapazität verbunden ist.

Der Erfindung liegt die Aufgabe zugrunde, Kurzzeit-Therapien transienter funktioneller Herz-Kreislauf Symptomatiken mit Betablockern zu ermöglichen und die Therapie funktioneller kardiovasculärer Syndrome insgesamt zu verbessern.

Diese Aufgabe wird erfindungsmäßig gelöst durch Verwendung einer Zusammensetzung zur Herstellung eines Medikaments für eine Kurzzeit-Therapie transienter funktioneller Herz-Kreislauf Symptome mit Betablockern, wobei die Zusammensetzung in oraler Arzneiform mit hydrophilem Betablocker vorliegt, die für den jeweils eingesetzten spezifischen Betablocker im Körper nur solche grenzwertig wirksamen Wirkstoff-Konzentrationen erzeugt, mit denen transiente adrenerge Stimulationseffekte klinisch wirksam verringert werden, ohne daß klinisch wirksame Änderungen der Herz-Kreislaufwerte unter Ruhebedingungen erzeugt werden, wobei die Arzneiform als Einzeldosis in Dosierintervallen von 24 bis 52 Stunden verabreicht wird und 10 % bis 50 % der gegenwärtig eingesetzten niedrigsten oralen therapeutischen Dosis des spezifischen Betablockers enthält, die bei dessen Anwendung in chronischen Therapien gegeben wird, mit der Maßgabe, daß eine Kurzzeit-Therapie ventrikulärer Tachycardien in Verbindung mit ventrikulären Arrhythmien ausgeschlossen ist.

Um für diese grenzwertig wirksamen Arzneiformen mit Betablockern die Therapiemöglichkeiten zu erweitern, wird, als neue medizinische Indikation für Betablocker, die primäre Therapie von Schlafstörungen im Rahmen funktioneller Syndrome eingesetzt, wobei hierzu insbesondere auch die Schlafstörungen im Rahmen postmenopausaler Symtomatiken einbezogen sind.

Um diese Therapie auch mit Einsatz einfacher zu erreichender technischer Maßnahmen zu erlangen, werden die Betablocker-Arzneiformen in der Form dargestellt, daß sie in solchen Verpackungen eingesetzt werden, die dazu vorgesehen sind, grenzwertige Betablockerkonzentrationen zur Behandlung vegetativer Symptomatiken zu erreichen, insbesondere mit der Aufnahme und Gestaltungsmerkmalen von Einzeldosierungen, die jeweils einen therapeutischen Effekt für mehrere Tage bewirken.

Mit den hydrophilen Betablockern wird für die Therapie der Anteil von direkten zentralnervösen Effekten verringert. Vorzugsweise werden Atenolol, Nadolol und Sotalol eingesetzt.

Die mit der Erfindung erzielten Vorteile bestehen insbesondere darin, daß mit diesen grenzwertig wirksamen Arzneiformen die Betablockertherapie vegetativer Symptomatiken und Syndrome therapeutisch sicherer als auch deren therapeutisches Anwendungsspektrum erweitert werden kann. So ist die Anwendung von Betablockern, z.B. zur Therapie von Schlafstörungen bei vegetativen Syndromen, mit den bisherigen Arzneiformen für Betablocker bisher nicht möglich.

Durch die grenzwertig wirksamen Arzneiformen mit Betablockern lässt sich insbesondere auch der Einsatz von zentralnervös wirkenden Therapien mit Tranquilizern und Barbituraten, die zahlreiche unerwünschte Wirkungen und Überhangeffekte beinhalten, erheblich senken. Auch das diesbezügliche Potential an Abhängigkeitsrisiken mit diesen Stoffen kann eingeschränkt werden.

Gegenüber üblichen oralen Arzneiformen mit Betablockern eröffnen die grenzwertig wirksamen Arzneiformen mit hydrophilen Betablockern auch die Möglichkeit, bei transienten vegetativen Symptomatiken Kurzzeit-Therapien ohne Gefahr kardiovaskulärer Absetzphänomene durchzuführen. So erzeugen die grenzwertig wirksamen Arzneiformen mit hydrophilen Betablockern nur eine Reduktion von adrenerg stimulierten Reaktionen, aber keine persistierenden Effekte auf basale Herz-Kreislauf-Ruhewerte, wie es bei üblichen Arzneiformen mit Betablockern der Fall ist.

Insbesondere entfällt hierdurch gegenüber üblichen Arzneiformen mit Betablockern der Zwang, auch bei der Therapie transient vegetativer Symptomatiken und Syndrome die Applikationsphasen der Betablocker länger auszudehnen als dies therapeutisch nötig ist.

Insgesamt wird mit den grenzwertig wirksamen Arzneiformen mit Betablockern auch, gegenüber den derzeitigen Arzneiformen mit Betablockern, die therapeutische Nutzen/Risiko Relation in der Behandlung vegetativer Symptomatiken erheblich verbessert, z.B. u.a. bezüglich der Leistungsfähigkeit und Lebensqualität der Patienten, bezüglich unerwünschter Wirkungen auf Lipid- und Glukose-Stoffwechsel, bei Begleitproblematiken wie eingeschränkter Nierenfunktion bei älteren Patienten und auch bei der Therapie von kindlichen Patienten.

Die oralen Dosen für vegetative Symptome liegen in einem Bereich von 10 % bis 50 % der gegenwärtig niedrigsten verabreichten Einzeldosis, wie sie bei chronischer Anwendung des jeweiligen Betablockers verabreicht wird. Somit kann die orale Einseldosis für Atenolol z.B. in einem Bereich von 2,5 bis 12,5 mg, für Nadolol in einem Bereich von 6 bis 30 mg, und für Sotalol in einem Bereich von 8 bis 40 mg liegen. Diese Einzeldosen werden in Dosierintervallen über 24 bis 52 Stunden verabreicht, um die vegetativen Symptome zu behandeln.

Verschiedene Dosen derselben oralen Arzneiform mit demselben Betablocker werden dosis-bezogen absorbiert und erzeugen eindeutige Dosis-Konzentrations-Beziehungen, so daß direkte Vergleiche möglich sind. Die Dauer und das Ausmaß der Wirkung des Betablockers ist demnach von der Dosis und der biologischen Affinität des Rezeptors abhängig. Die grenzwertig wirksamen Arzneiformen mit Betablockern können daher analog zu den auf dem pharmazeutischen Gebiet konventionellen Arzneiformen gewonnen werden. Die Herstellung einer grenzwertig wirksamen Arzneiform für orale Anwendungen beispielsweise entspricht der gleichen Prozedur wie die einer konventionellen oralen Arzneiform, nur daß die oralen grenzwertig wirksamen Arzneiformen lediglich Dosen in einem Bereich von z.B. 10 bis 50 % der Dosen enthalten, die bei der entsprechenden gewöhnlichen oralen Betablocker-Arzneiform verwendet werden, wenn diese für die konventionelle orale Anwendung zur chronischen Therapie in manifestierten Krankheiten verabreicht werden.

Zur Kurzzeit-Therapie transienter funktioneller Herz-Kreislauf Symptomatiken werden grenzwertig wirksame Arzneiformen mit hydrophilen Betablockern eingesetzt, die im Körper nur solche Wirkstoffkonzentrationen erzeugen, die für den jeweils eingesetzten spezifischen Betablocker keine signifikanten Anderungen der physiologischen Werte im Herz-Kreislauf-System unter Ruhebedingungen erzeugen und adrenerg induzierte transiente stinulatonseffekte signifikant reduzieren, wobei dies orale Arzneiformen sind. Hierdurch wird eine differenzierte Therapie funktioneller symptomatiken möglich, die nicht mit den für Langzeitherapien konzipierten, üblichen Arzneiformen mit Betablockern gegeben ist. Hierdurch werden sowohl die Lebensqualität wie auch die Nutzen-Risiko Relation der Betablocker verbessert. Die Therapiedauer muss auch nicht über das symptomatisch erforderliche Ausmass ausgedehnt werden, da keine Rebound-Gefahr nach Absetzen besteht. Als weitere und neue Indikation wird hiermit auch die Kurzzeit-Anwendung zur primären Therapie von Schlafstörungen im Rahmen vegetativer Syndrome, insbesondere auch im Rahmen postmenopausaler Symptomatiken, ermöglicht.

## Patentansprüche

1. Verwendung einer Zusammensetzung zur Herstellung eines Medikaments für eine Kurzzeit-Therapie transienter funktioneller Herz-Kreislauf-Symptome mit Betablockern, wobei die Zusammensetzung in oraler Arzneiform mit hydrophilem Betablocker vorliegt, die für den jeweils eingesetzten spezifischen Betablocker im Körper nur solche grenzwertig wirksamen Wirkstoff-Konzentrationen erzeugt, mit denen transiente adrenerge Stimulationseffekte klinisch wirksam verringert werden, ohne daß klinisch wirksame Änderungen der Herz-Kreislaufwerte unter Ruhebedingungen erzeugt werden, wobei die Arzneiform als Einzeldosis in Dosierintervallen von 24 - 52 h verabreicht wird in Mengen von 10 % bis 50 % der gegenwärtig eingesetzten niedrigsten oralen therapeutischen Einzeldosis des spezifischen Betablockers, die bei dessen Anwendung in chronischen Therapien gegeben wird, mit der Maßgabe, daß eine Kurzzeit-Therapie ventrikulärer Tachycardien in Verbindung mit ventrikulären Arrhythmien ausgeschlossen ist.

2. Verwendung gemäß Anspruch 1 zur Behandlung von Schlafstörungen im Rahmen funktioneller Syndrome, die insbesondere auch die Anwendung bei Schlafstörungen im Rahmen postmenopausaler Symptomatiken einbezieht.

3. Verwendung gemäß Anspruch 1, wobei die Arzneiformen in solchen Verpackungen eingesetzt werden, die dazu vorgesehen sind grenzwertige Betablockerkonzentrationen zur Behandlung vegetativer Symptomatiken zu erreichen, insbesondere auch mit der Aufnahme und Gestaltungsmerkmalen von Einzeldosierungen, die jeweils einen therapeutischen Effekt für mehrere Tage bewirken.

4. Verwendung gemäß Anspruch 1, wobei als hydrophiler Betablocker Atenolol, Nadolol oder Sotalol eingesetzt wird.

## Claims

1. Use of a composition for the production of a medicament for a short-term therapy of transient functional cardiovascular symptoms with beta-blockers, the composition being present in oral pharmaceutical form with a hydrophilic beta-blocker, which, for the specific beta-blocker employed in each case in the body, only produces those active compound concentrations having threshold activity with which transient adrenergic stimulation effects can be effectively clinically decreased without clinically effective alterations of the cardiovascular values being produced under resting conditions, the pharmaceutical form being administered as a single dose at dose intervals of 24 - 52 h in amounts of 10% to 50% of the currently employed lowest oral therapeutic single dose of the specific beta-blocker which is given during use thereof in chronic therapies, with the proviso that a short-term therapy of ventricular tachycardias in connection with ventricular arrhythmias is excluded.

2. Use according to Claim 1 for the treatment of sleep disorders in the context of functional syndromes, which in particular also includes use in sleep disorders in the course of post-menopausal symptomatologies.

3. Use according to Claim 1, the pharmaceutical forms being employed in those packs which are intended to achieve threshold beta-blocker concentrations for the treatment of vegetative symptomatologies, in particular also with the absorption and design features of single doses which in each case cause a therapeutic effect for a number of days.

4. Use according to Claim 1, the hydrophilic beta-blocker employed being atenolol, nadolol or sotalol.

## Revendications

1. Utilisation d'une composition pour la préparation d'un médicament destiné au traitement à court terme des symptômes cardiovasculaires fonctionnels transitoires par les bêta-bloquants, la composition se présentant sous une forme médicamenteuse orale contenant un bêta-bloquant hydrophile qui ne produit, pour le bêta-bloquant spécifique utilisé, que des concentrations intracorporelles de principe actif efficaces à des doses minimales, permettant de diminuer cliniquement de façon efficace les effets de stimulation adrénergique transitoires, sans provoquer de modifications cliniques efficaces des valeurs cardiovasculaires dans des conditions de repos, la forme médicamenteuse étant administrée en tant que dose unique sur des intervalles posologiques de 24 à 52 h en des quantités de 10 à 50 % de la dose unique thérapeutique orale la plus faible du bêta-bloquant spécifique actuellement utilisée, administrée lors de son application dans les traitements chroniques, à la condition que soit exclu un traitement à court terme des tachycardies ventriculaires liées à des arythmies ventriculaires.

2. Utilisation selon la revendication 1, pour le traitement des insomnies dans le cadre de syndromes fonctionnels, comprenant notamment également l'utilisation lors d'insomnies dans le cadre de symptômes post-ménopausiques.

3. Utilisation selon la revendication 1, les formes médicamenteuses étant utilisées dans des emballages prévus pour atteindre des concentrations de bêta-bloquant minimales pour le traitement des symptômes végétatifs, notamment avec l'absorption et les caractéristiques de configuration des doses uniques provoquant un effet thérapeutique pendant plusieurs jours.

4. Utilisation selon la revendication 1, le bêta-bloquant hydrophile utilisé étant l'aténolol, le nadolol ou le sotalol.
